(19) Europäisches Patentamt

European Patent Office

Office européen des brevets

(11) Publication number: **0 221 580 B1**

## EUROPEAN PATENT SPECIFICATION

(45) Date of publication of patent specification: 07.08.91    (51) Int. Cl.⁵: **A61L 2/06**

(21) Application number: 86201584.9

(22) Date of filing: 16.09.86

(54) **Equipment for sterilization of mineral-wool.**

(30) Priority: 05.11.85 NL 8503027

(43) Date of publication of application:
13.05.87 Bulletin 87/20

(45) Publication of the grant of the patent:
07.08.91 Bulletin 91/32

(84) Designated Contracting States:
**BE DE FR NL**

(56) References cited:
**AT-B- 357 268**
**DE-C- 867 289**
**DE-C- 898 351**

(73) Proprietor: **Barel, Marten Dirk E.**
**Roskam 22**
**NL-5505 JJ Veldhoven(NL)**

(72) Inventor: **Barel, Marten Dirk E.**
**Roskam 22**
**NL-5505 JJ Veldhoven(NL)**

(74) Representative: **Hoorweg, Petrus Nicolaas et al**
**OCTROOIBUREAU ARNOLD & SIEDSMA**
**Sweelinckplein 1**
**NL-2517 GK The Hague(NL)**

## Description

The invention relates to a device for sterilizing stackable materials, e.g. bars of mineral wool for hydroculture, comprising a chest fitted with a door and means to circulate steam from the top to the bottom through the material placed in the chest and fan means for creating a sub-atmospheric pressure in the interior of the chest.

Such a device is already disclosed in DE-C-898351. An important disadvantage is that the useful effect is affected highly disadvantageously due to the fact that the steam will chiefly take the line of the least resistance, so that it will not flow trough, but past the materials placed in the chest. The invention aims to improve the above.

Substantially the above has been improved in that the exhaust side of the fan means is connected to the spaces between the steam chest walls and a flexible inner wall adapted to engage the outline of the stacked material to be sterilized when creating reduced pressure, wherein the top edge protions of said inner wall are provided with pressure relief holes in such a manner that the steam is forced to pass through the material to be sterilized.

On account of this measure a difference in pressure is established between the spaces between the flexible wall and the steam chest walls on the one hand and the space in which the material to be sterilized is placed on the other hand, and, moreover, a large part of the thermical energy stored in the steam is conserved.

Preferably a steam branch pipe, being branched from the vertical steam conduit, is connected to the pressure passage of the fan.

On account of this measure the duration of the treatment is shortened.

In order to be able to open the door and yet to fit it with a flexible inner wall portion the measure has been applied that the flexible inner wall is made double-walled on the location of the door hinges.

The installation in question can be transportable and if so, is characterized in that the required steam is generated in a boiler being mounted on a truck, and in that two adjacent chests are mounted on a trailer which also accomodates a fork lift truck that can transport the materials to be sterilized or the sterilized materials respectively, being stacked on a pallet, to or from the steam chests respectively.

The invention will be elucidated below on the basis of the drawing, in which, by way of example, an embodiment of an installation according to the invention is represented. In the drawing:

fig. 1 shows a view of a chest with an open door,

fig. 2 shows a horizontal section along line II-II of the chest of fig. 1,

fig. 3 shows a vertical section along line III-III through the chest of fig. 2,

fig. 4 shows a detail of the flexible inner wall, and

fig. 5 shows a complete view of a road train with an installation in which two chests according to the invention have been incorporated.

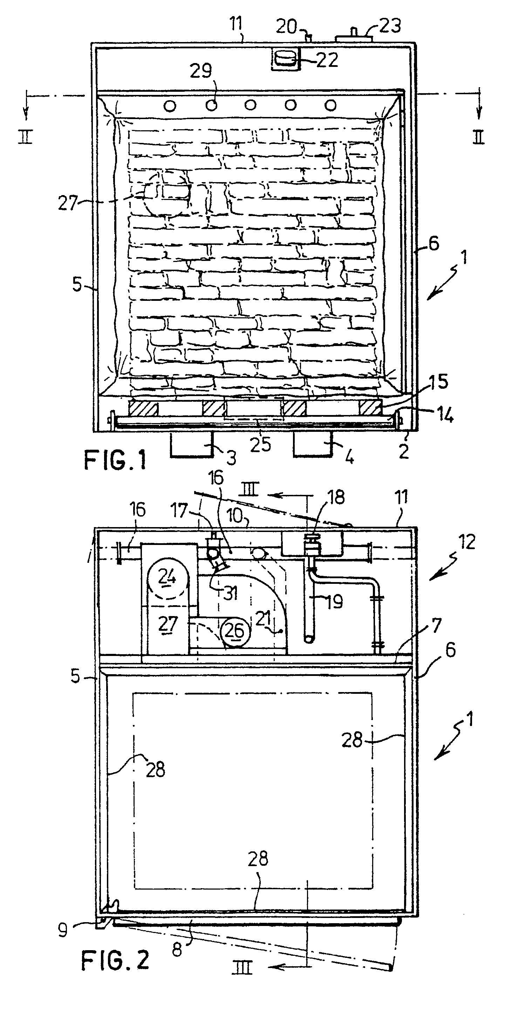

The steam chest 1 of the installation represented in figures 1-3 comprises a bottom 2 placed on two longitudinal girders 3 and 4, two side walls 5 and 6, a back wall 7, and a door 8 which is connected, by hinges 9, to a side wall 5 and can be considered the front wall. The door 8 has to be able to withstand, as far as the fastening and construction of its pivots (9) and (not visible) lock are concerned, the pressure that can prevail in the interior of the steam chest. Preferably additional locking bars with espagnolette fasteners are provided to hold the upper and lower edges of the door as securely as possible against the remaining walls of the chest.

The bottom 2 and the side walls 5 and 6 extend beyond the back wall 7 and define, together with an end wall 11 comprising a small door 10, an apparatus box 12. The steam chest 1 and the apparatus chest 12 are covered on top by a joint roof 13.

On the floor portion 2 in the steam chest 1 a roller track is applied, so that pallets 15 with blocks of mineral wool stacked onto it that can be supplied and discharged by means of a fork lift truck, can be easily transported into and out of the steam chest. Protection means (not shown) have been applied on the roller track 14 along the side walls 5 and 6 which have to prevent the flexible wall 28, yet to be discussed, from being damaged. There are about six sizes of blocks of mineral wool, but all of them can be stacked securely onto a so-called Europallet, the outer walls being as flat as possible. It is also possible to sterilize mineral wool in polypropyl basins, and of course to sterilize other objects than those used in horticulture; the invention can, in principle, be applied to any stackable material to be sterilized.

Through the apparatus box 12 parallel to the end wall 11 extends a horizontal steam conduit 16, the one end of which can be connected to a source of compressed steam. If a plurality of steam chests 1 is disposed in series, the other end of the horizontal steam conduit 16 can be connected to the steam conduit 16 of the next steam chest. The pressure in the steam conduit 16 can be read from a manometer 18 and as a safe-guard branch pipe 19 has been applied extending to a pressure relief valve 20 in the roof 13. A vertical steam steam pipe 21 is branched upwards from the horizontal steam conduit 16, said steam pipe comprising a steam inlet cock 17. In order to operate the cock 17 from the big door 8, a simple chain transmission has been applied extending to an operation shaft passing under the steam chest perpendicularly to the horizontal steam conduit 16. The steam can enter the steam chest via the conduit 21 extending to an upper steam inlet 22 and again a pressure relief valve 23 has been applied in the roof 13. Thus far the facts on the steam circuit.

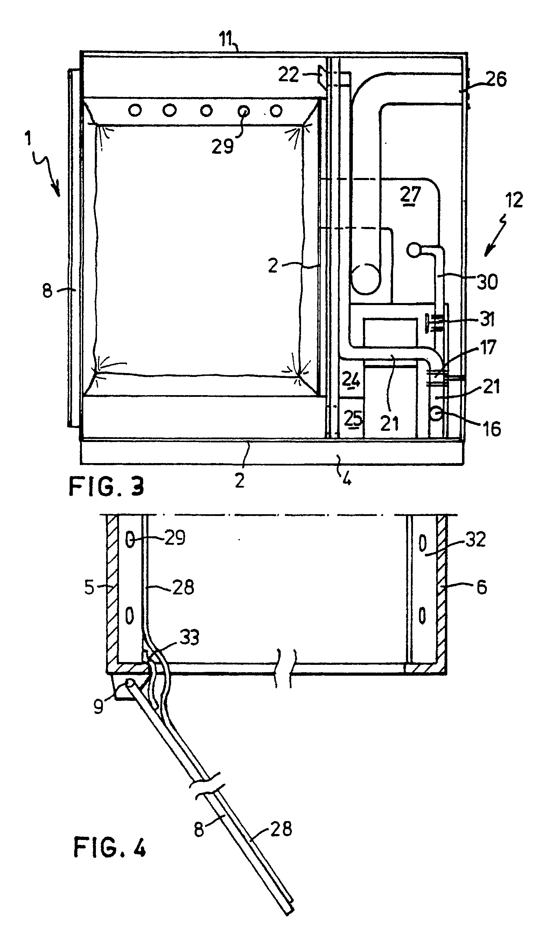

However, before the steam is admitted into the steam chest 1, first a sub-atmospheric pressure is created in the chest. This is performed by means of a fan 24 with intake opening 25. In fig. 2 and 3 one can also see an intake passage 26 for cooling the fan motor (not represented).

The pressure side of the fan 24 is connected, through a pressure passage 27, with spaces being defined by the side walls 5 and 6, the back wall 7 and the door 8 on the one hand and by a flexible inner wall 28, being connected to said walls and the door hermetically sealed along its edges. The top edges of the flexible inner wall 28 are enclosed in obliquely protruding suspension strips with pressure relief holes 29. A likely material for the flexible wall is in the first place a so-called steam cloth, which is also used for ground steaming. This material is fairly rigid at ambient temperature and able to withstand the operating temperature of the steam chest, while becoming more flexible at increasing temperatures.

Past the steam inlet cock 17 a steam branch pipe 30, being connected to the pressure passage 27 through a steam branch cock 31 of its own, is branched off from the vertical steam conduit 21.

Fig. 4 shows a horizontal section through the steam chest 1 at the level of the roof 13, so as to elucidate a double-walled embodiment of the flexible inner wall 28 secured to the suspension strip 32 with pressure relief holes 29 at the location of hinge 9. The internal slit of the hinge 9 is covered by a strip 33 of the same material as the inner wall 28 and this strip is connected in vertical direction to the side wall 5 and door 8, and in horizontal direction to the opposite edge portions of the flexible inner wall 28, creating, so to speak, a tunnel through which the excess pressure generated by a fan can also reach the space between the door and the flexible inner wall 28.

The working of the steam chest 1 is as follows: after placing a pallet 15 with compact and smoothly stacked mineral wool and closing the door 8, all the air is sucked away from under the pallet by means of the fan 24. Then this air is circulated through the mineral wool via pressure relief holes 29. Furthermore steam is blown over the pallet at 170° and, on account of the sub-atmospheric pressure under the pallet, drawn through the material to be sterilized very quickly. A portion of the steam, adjustable by the steam branch cock 31, ends up directly behind the flexible walls, so that they reach the required temperature sooner and thus fit properly sooner than would be the case if the detour through the steam chest itself had been made. Within about 2 minutes the mineral wool is at 100° C, which is sufficient to kill all the germs and to use the material in the green-house for another year. The temperature of the mineral wool can easily be measured by sticking a thermo-couple into it. This repeated use instead of annual replacement renders a huge relief of the environmental burden, since mineral wool is neither degradable nor processable. In the above-described manner, the mineral wool can be used for about seven years.

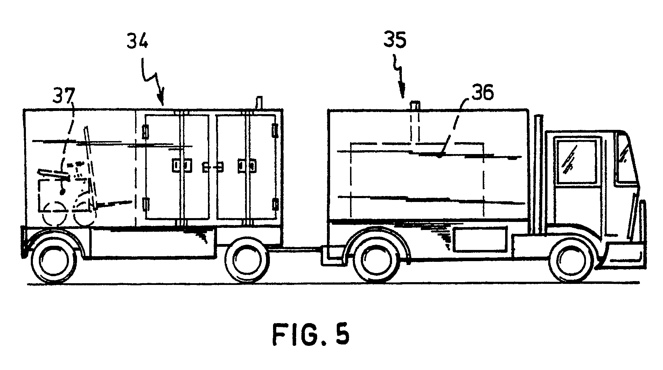

It appears that an above-described steam chest can be worked extremely efficiently by placing two chests on a trailer 34, in which chest a sub-atmospheric pressure of about 0.3 atm can be created by means of the pertaining fans. Then they cooperate with a boiler 36 placed on a truck 35 which supplies an excess pressure of max. 0.5 ato at a capacity of about 800 kg steam/h. Such a train is represented in fig. 5 and the trailer also accomodates a fork lift truck 37 with which the pallets can be efficiently transported. The handling capacity of the train represented here is about 15 to 18 pallets per hour.

However, other embodiments than the train represented in fig. 5 are also within the scope of the claims, one can particularly think of stationary installations for other materials to be sterilized, if only there is at least one flexible inner wall which is adapted to engage the outline of the stacked material to be sterilized, when creating reduced pressure, in such a manner that the steam is forced to pass through the material to be sterilized.

## Claims

1. A device for sterilizing stackable materials, e.g. bars of mineral wool for hydroculture, comprising a chest (1) fitted with a door (8) and means to circulate steam (16-21) from the top to the bottom through the material placed in the chest and fan means (24) for creating a sub-atmospheric pressure in the interior of the chest, characterized in that the exhaust side of the fan means (24) is connected to the spaces between the steam chest walls (5-8) and a flexible inner wall (28) adapted to engage the outline of the stacked material to be-sterilized when creating reduced pressure, wherein the top edge protions of said inner wall are provided with pressure relief holes (29) in such a manner that the steam is forced to pass through the material to be sterilized.

2. A device according to claim 1, characterized in that a steam branch pipe (30), being branched from the vertical steam conduit (21), is connected to the pressure passage (27) of the fan (24).

3. A device according to any of the claims 1 or 2, characterized in that the flexible inner wall (28) is made double-walled on the location of the door (8) hinges (9).

4. A device according to any of the preceding claims, characterized in that the required steam is generated in a boiler being mounted on a truck, and in that two adjacant chests are mounted on a trailer which also accomodates a fork lift truck that can transport the materials to be sterilized or the sterilized materials respectively, being stacked on a pallet, to or from the steam chests respectively.

## Revendications

1. Dispositif pour stériliser des matériaux empilables, par exemple des barres de laine minérale pour l'hydroculture, comprenant une caisse (1) équipée d'une porte (8) et de moyens (16,21) pour faire circuler de la vapeur depuis la partie supérieure jusqu'à la partie inférieure à travers le matériau placé dans la caisse, et une soufflerie 24 pour engendrer à l'intérieur de la caisse une pression inférieure à la pression atmosphérique, caractérisé en ce que le côté refoulement de la soufflerie (24) est relié à des espaces situés entre les parois (5,8) de la caisse à vapeur et une paroi interne souple (28) adaptée pour venir en contact avec le contour extérieur du matériau empilé à stériliser lorsqu' on engendre une pression réduite, les parties des bords supérieurs de ladite paroi interne étant pourvues de trous (29) de décharge de pression de manière que la vapeur soit forcée de traverser le matériau à stériliser.

2. Dispositif suivant la revendication 1, caractérisé en ce qu'un tuyau (30) de dérivation de vapeur, s'étendant à partir d'un conduit vertical de vapeur (21), est relié au conduit de pression (27) de la soufflerie (24).

3. Dispositif suivant l'une quelconque des revendications 1 ou 2, caractérisé en ce que la paroi interne souple (28) est double à l'emplacement des charnières (9) de la porte (8).

4. Dispositif suivant l'une quelconque des revendications précédentes, cactérisé en ce que la vapeur nécessaire est engendrée dans une chaudière mise en place sur un camion, et en ce que deux caisses adjacentes sont mises en place sur une remorque qui reçoit également un chariot élévateur à fourche qui peut transporter les matériaux à stériliser ou les matériaux stérilisés, qui sont empilés sur une palette, respectivement à l'intérieur et à l'extérieur des caisses à vapeur.

## Patentansprüche

1. Vorrichtung zur Sterilisation aufzuschichtenden Materials, beispielsweise von Riegeln aus Steinwolle für die Hydrokultur, mit einem Kasten (1), der mit einer Türe (8) versehen ist sowie Einrichtungen zum Zirkulieren von Dampf (16-21) von oben nach unten durch das in dem Kasten angeordnete Material, und mit einer Gebläseeinrichtung (24) zur Erzeugung eines subatmosphärischen Druckes im Inneren des Kasten, dadurch gekennzeichnet, daß die Abdampfseite der Gebläseeinrichtung (24) mit den Räumen zwischen den Dampfkastenwandungen (5-8) verbunden ist und eine flexible Innenwand (28) derart angepaßt ist, daß sie mit dem Umriß des zu sterilisierenden Materiales in Eingriff steht, wenn ein reduzierter Druck erzeugt wird, wobei die Oberkantenabschnitte der Innenwand mit Druckentlastungslöchern (29) derart versehen sind, daß der Dampf gezwungen wird, durch daß zu sterilisierende Material zu dringen.

2. Vorrichtung nach Anspruch 1, gekennzeichnet durch ein Dampfabzweigrohr (30), das von der vertikalen Dampfleitung (21) abzweigt und mit dem Druckkanal (27) des Gebläses (24) verbunden ist.

3. Vorrichtung nach einem der Ansprüche 1 oder 2, dadurch gekennzeichnet, daß die flexible Innenwand (28) am Ort der Scharniere (9) der Türe (8) doppelwandig ausgebildet ist.

4. Vorrichtung nach einem der vorstehenden Ansprüche, dadurch gekennzeichnet, daß der erforderliche Dampf in einem auf einem Lastwagen montierten Boiler erzeugt wird und daß zwei aneinander anschließende Kästen auf einen Anhänger montiert sind, der ebenfalls ein Gabelstaplerfahrzeug beinhaltet, welches die zu sterilisierenden Materialien oder die sterilisierten Materialien, die auf einer Palette geschichtet sind zu oder von den Dampfkästen weg transportiert.

FIG.1

FIG.2

FIG. 3

FIG. 4

FIG. 5